# EUROPÄISCHE PATENTANMELDUNG

(11) **EP 4 410 223 A2**
(43) Veröffentlichungstag der Anmeldung: **07.08.2024**
(21) Anmeldenummer: 24154446.9
(22) Anmeldetag: 29.01.2024
(51) Int. Cl.: A61B 17/16, A61B 17/32, A61B 90/00

(54) **MEDIZINISCHE AUFNAHMEVORRICHTUNG UND MEDIZINISCHES HANDSTÜCK ZUM ERMITTELN EINES VERSCHLEISSGRADS EINES MEDIZINISCHEN WERKZEUGS**

(30) Priorität: 02.02.2023 DE 102023102615
(71) Anmelder: Aesculap AG, 78532 Tuttlingen (DE)
(72) Erfinder: BUERK, Andre, 78056 Villingen-Schwenningen (DE); VIERTEL, Bjoern, 52074 Aachen (DE)
(74) Vertreter: Winter, Brandl - Partnerschaft mbB

(57) **Zusammenfassung**

Die vorliegende Offenbarung betrifft eine medizinische Aufnahmevorrichtung zur Aufnahme einer äußeren Belastung eines chirurgischen Schaftwerkzeugs (9) während eines chirurgischen Behandlungsvorgangs, aufweisend:
- einen Lagerungsschaft (3) für eine oder einer chirurgischen Behandlungsvorrichtung (1), der zur lagernden Aufnahme des chirurgischen Schaftwerkzeugs (9) ausgebildet ist,
- zumindest einen elastisch verformbaren Lagerungsschaftabschnitt (SA), der eine bestimmte, vorzugsweise vorbestimmte Biegeelastizität hat und
- eine Erfassungsvorrichtung, die dafür ausgebildet ist, einen Biegeverformungszustand und/ oder Biegeverformungsbewegung und/ oder einen Grad der Biegeverformung des Lagerungsschaftabschnitts (SA) infolge der äußeren Belastung zu erfassen. Weiterhin betrifft die Offenbarung ein medizinisches Handstück (1) mit einem Lagerungsschaft (SA), ein System aus dem medizinischen Handstück (1) und einer Auswerteeinheit (69) und ein Verfahren zum Ermitteln eines Verschleißgrads eines medizinischen Werkzeugs (9).

## Beschreibung

### Technisches Gebiet

Die vorliegende Offenbarung betrifft eine medizinische Aufnahme- bzw. Detektionsvorrichtung, ein medizinisches Handstück mit einem Lagerungsschaft, ein System aus dem medizinischen Handstück und einer Auswerteeinheit und ein Verfahren zum Ermitteln eines Verschleißgrads eines medizinischen Werkzeugs.

### Hintergrund der Offenbarung

Rotatorisch und/ oder oszillierend angetriebene Werkzeuge/ Schaftwerkzeuge insbesondere mit spanabhebenden, polierenden und/oder schneidenden Effektoren (Werkzeugköpfen) finden in weiten Bereichen der Medizin tagtäglich Anwendung. So werden derartige Werkzeuge beispielsweise in chirurgischen Anwendungen an Knochen und/ oder Knorpelgewebe als Abrasivwerkzeuge eingesetzt.

Derartige Werkzeuge unterliegen während eines Einsatzes, d.h. während eines chirurgischen Behandlungsvorgangs einem Verschleiß, welcher sich in einer reduzierten Effektor-, insbesondere Schneidleistung äußert bzw. in einer reduzierten Schneidleistung des Werkzeugs resultiert. Die reduzierte Schneidleistung hat zur Folge, dass höhere Temperaturen an einem Eingriffsort des Werkzeugs entstehen können, welche den Patienten schädigen können. Auch ist zu beobachten, dass beispielsweise ein Chirurg die zunehmend sich verringernde Effektor-/Schneidleistung durch Erhöhen seines eigenen Kraftaufwands zu kompensieren versucht, was wiederum dazu führt, dass insbesondere Lagerungsbauteile am chirurgischen Instrument, in welches das Werkzeug wahlweise/temporär eingesetzt ist, ebenfalls stärker belastet werden. Auch elektrische Antriebe müssen dann mehr Leistung zur Betätigung des Werkzeugs aufbringen, was neben der erhöhten Abnutzung auch zu verstärkter Wärmeentwicklung am Antrieb führt.

### Stand der Technik

Stand heute gibt es keine andere Möglichkeit, den Verschleiß eines Werkzeugs im Behandlungsprozess zu erkennen, als analog durch den erfahrenen Anwender/ Behandler, der eine Veränderung der Schneidleistung erkennen und das Werkzeug austauschen muss.

Da es sich bei dem Werkzeugverschleiß und damit bei der Reduktion der Schneidleistung um einen kontinuierlichen Prozess handelt, der über den Behandlungsprozess fortschreitet, ist es für den Anwender besonders schwierig, einen perfekten Werkzeugwechselzeitpunkt rein basierend auf seiner Erfahrung zu erkennen und/ oder zu bestimmen. Auch die Erfassung der Gesamtbenutzungszeit eines Werkzeugs führt nicht grundsätzlich zu einem befriedigenden Ergebnis, da hierdurch die auf das Werkzeug aufgebrachte, individuell auf einen Chirurgen zurückgehende Benutzerkraft entweder gar nicht, oder ggf. nur über das Antriebsmoment näherungsweise berücksichtigt wird.

Dies hat zur Folge, dass Werkzeuge entweder zu früh gewechselt werden, was zu erhöhten Kosten führt, oder, dass Werkzeuge zu spät gewechselt werden, was ein Operationsergebnis beeinträchtigen und ggf. sogar eine Patientensicherheit gefährden kann.

### Zusammenfassung der Offenbarung

Aufgabe der vorliegenden Offenbarung ist es, die Nachteile aus dem Stand der Technik zu beheben oder zumindest zu reduzieren.

Konkret ist es Aufgabe der vorliegenden Offenbarung, eine Möglichkeit bereitzustellen, einen Verschleiß eines medizinischen Werkzeugs im Behandlungsprozess zu detektieren und eine optimale Werkzeugstandzeit/ Werkzeugnutzungszeit zu erreichen und eine Patientensicherheit während des Behandlungsvorgangs zu erhöhen.

Diese Aufgabe wird gelöst durch eine medizinische Aufnahmevorrichtung nach Anspruch 1. Weiterhin wird die Aufgabe gelöst durch ein medizinisches Handstück, ein System und ein Verfahren nach einem der nebengeordneten Ansprüche.

Konkret wird die Aufgabe gelöst durch eine medizinische Detektions-/ Aufnahmevorrichtung zur Aufnahme einer äußeren (beispielsweise von einem Chirurgen oder einer Robotik aufgebrachten) Belastung eines (in ein chirurgisches / medizinisches Handinstrument oder eine Chirurgie-Robotik eingesetzten) chirurgischen Schaftwerkzeugs während eines chirurgischen Behandlungsvorgangs / einer auf das chirurgische Schaftwerkzeug wirkenden Kraft. Die medizinische Aufnahmevorrichtung beinhaltet einen Lagerungsschaft für eine oder einer chirurgischen Behandlungsvorrichtung (chirurgisches / medizinisches Handinstrument oder Chirurgie-Robotik), der zur lagernden Aufnahme / Abstützung des chirurgischen Schaftwerkzeugs ausgebildet ist. Weiterhin beinhaltet die medizinische Detektions-/Aufnahmevorrichtung zumindest einen elastisch verformbaren Lagerungsschaftabschnitt oder Schaftteil, der eine bestimmte, vorzugsweise vorbestimmte/vorbestimmbare Biegeelastizität hat und eine Erfassungsvorrichtung (Sensorik), die dafür ausgebildet ist, einen Biegeverformungszustand und/oder eine Biegeverformungsbewegung und/ oder einen Grad der Biegeverformung des Lagerungsschaftabschnitts infolge der äußeren Belastung zu erfassen.

In anderen Worten wird die Aufgabe gelöst durch die medizinische Detektions-/ Aufnahmevorrichtung, welche insbesondere integriert in oder adaptiert an den Lagerungsschaft ist/wird. Der Lagerungsschaft ist vorgesehen und ausgebildet, an ein medizinisches Handstück/ Griffstück oder einen medizinischen Roboter zur Lagerung/Aufnahme/Abstützung des medizinischen Schaftwerkzeugs befestigt/ gekoppelt zu werden.

Der Lagerungsschaft weist im Wesentlichen eine rohrförmige Geometrie auf insbesondere mit einem Kupplungsstück an seinem proximalen Endabschnitt, mittels welchem der Lagerungsschaft auswechselbar mit dem medizinischen Handstück/ Griffstück oder dem medizinischen Roboter in Kupplungseingriff bringbar ist.

Die Detektions-/ Aufnahmevorrichtung (nachfolgend nur noch als Aufnahmevorrichtung bezeichnet) ist ausgebildet, eine äußere (vom Chirurgen oder Roboter aufgebrachte) Belastung, insbesondere in Form einer Radialkraft, welche während des chirurgischen Behandlungsvorgangs auf das Schaftwerkzeug wirkt, aufzunehmen/ zu erfassen/ zu detektieren. Der Lagerungsschaft ist ein Handstück- bzw. Roboter-seitiger Schaft, welcher den Werkzeugschaft des medizinischen Schaftwerkzeugs drehbar lagert und ggf. einen Drehmomentübertragungszug enthält, der vorgesehen und ausgebildet ist, eine Antriebskraft vom Handstück- oder Roboterseitigen Antrieb auf das medizinische Schaftwerkzeug zu übertragen. Das medizinische Schaftwerkzeug ist also ein Werkzeug, welches rotatorisch und/ oder oszillierend angetrieben wird/ antreibbar ist. Insbesondere kann es sich bei dem medizinischen Schaftwerkzeug um einen medizinischen Fräser oder einen medizinischen Bohrer handeln.

Die medizinische Aufnahmevorrichtung beinhaltet weiterhin den elastisch verformbaren Lagerungsschaftabschnitt, wobei der elastisch verformbare Lagerungsschaftabschnitt vorzugsweise als ein Längsabschnitt des Lagerungsschafts ausgebildet ist. Der elastisch verformbare Lagerungsschaftabschnitt weist eine bestimmte, vorzugsweise vorbestimmte Biegeelastizität auf, wobei die Biegeelastizität fix oder einstellbar sein kann. Unter elastisch verformbar ist vorzugsweise zu verstehen, dass der elastisch verformbare Lagerungsschaftabschnitt bei Wegnahme der äußeren Belastung in einen gestreckten/ geraden Ausgangszustand zurückfedert.

Zusätzlich beinhaltet die medizinische Aufnahmevorrichtung die Erfassungsvorrichtung, welche vorgesehen und ausgebildet ist, den Biegeverformungszustand und/ oder die Biegeverformungsbewegung und/ oder eine Ausprägung/ Stärke/ den Grad der Biegeverformung des Lagerungsschaftabschnitts aufzunehmen/ zu erfassen/ zu detektieren. Anders ausgedrückt ist die Erfassungsvorrichtung ausgebildet, zu detektieren, ob eine Biegeverformung auftritt, aufgetreten ist und / oder wie stark die Biegeverformung ist.

Eine derartige Aufnahmevorrichtung kann anhand des Biegeverformungszustands und/ oder der Biegeverformungsbewegung und/ oder der Ausprägung/ Stärke/ des Grads der Biegeverformung des Lagerungsschaftabschnitts ein Signal erzeugen, welches eine auf das Schaftwerkzeug wirkende (Radial-) Kraft während des Eingriffs/ während des chirurgischen Behandlungsvorgangs anzeigt. Die auf das Schaftwerkzeug wirkende Kraft steht in direktem Zusammenhang mit einem Verschleißzustand des Schaftwerkzeugs. Konkret weist eine höhere Kraft auf ein verschlissenes Schaftwerkzeug hin. Auf diese Weise kann die offenbarungsgemäße Aufnahmevorrichtung den Verschleißzustand des Schaftwerkzeugs aufnehmen/ erfassen/ detektieren und ein konkreter/ idealer Zeitpunkt für einen Werkzeugwechsel kann bestimmt werden. So kann eine maximale Werkzeugstandzeit erzielt werden, Verletzungsrisiken für einen Patienten reduziert werden und ein Behandler entlastet werden, da er nicht manuell/ haptisch/ optisch/ analog, basierend auf seiner Erfahrung, den Zeitpunkt für den Werkzeugwechsel bestimmen muss.

Die derartige Aufnahmevorrichtung ermöglicht eine permanente Erfassung von Kräften während des Behandlungsvorgangs, eine Warnmöglichkeit bei zu starkem Druck durch einen Bediener auf das Schaftwerkzeug während des chirurgischen Behandlungsvorgangs, automatische Erkennung von Verschleiß durch die permanente Erfassung der Kräfte, Hinweis auf den empfohlenen Werkzeugwechsel, Loggen/ Aufzeichnen einer Verschleißhistorie von wiederverwendbaren Werkzeugen, optional individuell werkzeugbezogen.

Bei einer integrierten Lösung wird die Aufgabe gelöst durch den Lagerungsschaft zur Aufnahme des rotatorisch und/ oder oszillierend angetriebenen medizinischen Schaftwerkzeugs an dem medizinischen Handstück und/ oder dem medizinischen Roboter, wobei der Lagerungsschaft zumindest einen elastisch verformbaren Lagerungsschaftabschnitt beinhaltet und in diesem Lagerungsschaftabschnitt zumindest ein Aufnehmer der zumindest einen Erfassungsvorrichtung angeordnet ist, wobei die Erfassungsvorrichtung vorgesehen und ausgebildet ist, die Biegeverformung und/ oder einen Grad der Biegeverformung des Lagerungsschafts zu detektieren.

Kern der Offenbarung ist demnach die Aufnahmevorrichtung, welche die Biegeverformung eines Lagerungsschaft mittels der Erfassungsvorrichtung erfasst und so eine äußere Belastung des chirurgischen Schaftwerkzeugs während des chirurgischen Behandlungsvorgangs aufnimmt.

In einem ersten Aspekt kann die Erfassungsvorrichtung zumindest einen Sensor, vorzugsweise ein Kraftsensor, insbesondere ein Piezosensor oder ein Force Sensing Resistor, beinhalten.

In anderen Worten kann die Erfassungsvorrichtung, welches die Signale, die die äußere Belastung des chirurgischen Schaftwerkzeugs während des chirurgischen Behandlungsvorgangs beschreibt detektiert, ein Sensor sein. Bevorzugt handelt es sich bei dem Sensor um einen Kraftsensor. Besonders geeignet kann ein Dehnmessstreifen oder eine Force Sensing Resistor/ Force Sensitive Resistor sein.

Durch einen derartigen Sensor kann das die äußere Belastung des medizinischen Schaftwerkzeugs angebende/ beschreibende Signal einfach, kostengünstig und wartungsarm erzeugt werden.

In einem weiteren Aspekt kann die Erfassungsvorrichtung zumindest zwei Sensoren aufweisen, wobei die zwei Sensoren orthogonal zueinander in dem Lagerungsschaft angeordnet sein können.

Durch eine derartige Anordnung von mehreren Sensoren orthogonal zueinander kann eine Richtung der auf das Schaftwerkzeug wirkenden Kraft zuverlässig detektiert werden.

In einem weiteren Aspekt kann der Lagerungsschaft Lagerstellen zur Lagerung des medizinischen Schaftwerkzeugs beinhalten und die Erfassungsvorrichtung kann, zumindest teilweise, zwischen den Lagerstellen angeordnet/ ausgebildet sein.

In anderen Worten kann der Lagerungsschaft Lagerstellen, welche als Kugellager, Kugellagerpaare, Rollenlager, Nadellager oder jede andere Art von Lagern, die ein rotatorisches und/ oder oszillierendes Lagern des Schaftwerkzeugs in dem Lagerungsschaft ermöglichen, beinhalten. Insbesondere kann der Lagerungsschaft zwei Lagerstellen beinhalten, die beabstandet zueinander in dem Lagerungsschaft ausgebildet sein können. Zwischen den Lagerstellen kann die Erfassungsvorrichtung oder Bestandteile der Erfassungsvorrichtung derart angeordnet sein, dass der Biegeverformungszustand und/ oder die Biegeverformungsbewegung und/ oder der Grad der Biegeverformung von der Erfassungsvorrichtung erfasst werden kann.

Durch eine derartige Anordnung der Erfassungsvorrichtung kann sichergestellt werden, dass eine erhöhte Gegenkraft auf/ für die distale bzw. einem Werkzeugkopf des Schaftwerkzeugs zugewandte Lagerstelle, welche durch den erhöhten Schaftwerkzeugverschleiß entsteht/ erzeugt wird, zuverlässig erfasst werden. Da sich das Schaftwerkzeug über beide Lagerstellen abstützt und über das entsprechende Biegemoment je nach Verschleiß des Schaftwerkzeugs ein Bereich zwischen den Lagerstellen unterschiedlich stark gedehnt bzw. gestaucht bzw. gebogen wird, kann die Erfassungsvorrichtung verschleißabhängige Signale generieren.

In einem weiteren Aspekt kann der Lagerungsschaft einen proximalen Kopplungsabschnitt zur Ankopplung des Schafts an das medizinische Handstück und/ oder den medizinischen Roboter beinhalten und Leiterbahnen aufweisen, welche vorgesehen und ausgebildet sind, die Signale der Erfassungsvorrichtung zu leiten. Die Leiterbahnen können zwischen der Erfassungsvorrichtung und dem Kopplungsabschnitt ausgebildet sein.

In anderen Worten können die von der Erfassungsvorrichtung generierten Signale, die den Biegeverformungszustand und/ oder die Biegeverformungsbewegung und/ oder den Grad der Biegeverformung abbilden, über die Leiterbahnen zu dem Kopplungsabschnitt geleitet werden.

Durch die Leiterbahnen kann das Signal zuverlässig und bauraumsparend an den Kopplungsabschnitt des Lagerungsschafts geleitet werden, welcher als eine Schnittstelle fungiert bzw. als eine Schnittstelle ausgebildet sein kann.

In einem weiteren Aspekt kann in dem elastisch verformbaren Lagerungsschaftabschnitt eine Innenhülse oder Zwischenhülse ausgebildet sein, welche die zumindest eine Erfassungsvorrichtung beinhalten kann.

In anderen Worten kann zwischen den Lagerstellen die Innenhülse oder Zwischenhülse ausgebildet sein. Die Hülse kann die Lager der Lagerstellen zueinander positionieren. Weiterhin kann die Hülse die Erfassungsvorrichtung oder Teile der Erfassungsvorrichtung beinhalten oder kontaktieren oder tragen.

Durch eine derartige Innenhülse oder Zwischenhülse kann eine Montierbarkeit verbessert werden.

In einem weiteren Aspekt kann die Innenhülse oder Zwischenhülse über zumindest einen, vorzugsweise zwei, Stift(e) mit dem Lagerungsschaft verbunden sein.

In einem weiteren Aspekt kann der elastisch verformbare Lagerungsschaftabschnitt von einer Schutzschicht ummantelt sein. Bei der Schutzschicht kann es sich beispielsweise um einen Schrumpfschlauch oder dergleichen handeln, der den Lagerungsschaftabschnitt vor Umwelteinflüssen wie beispielsweise Schmutzpartikeln oder flüssigen Medien schützt, welche die Biegeelastizität des Lagerungsschaftabschnitts und/ oder die Funktion des chirurgischen Schaftwerkzeugs beeinträchtigen können.

In einem Aspekt kann der Lagerungsschaft vollständig aus einem elastischen Material ausgebildet sein.

Der Lagerungsschaft kann seine Biegeelastizität über eine Längserstreckung des Lagerungsschafts variieren.

In einem weiteren Aspekt kann der elastisch verformbare Lagerungsschaftabschnitt Ausnehmungen, vorzugsweise in Form von Schlitzen oder einer Wandstärke reduzierenden Verjüngung, beinhalten.

In anderen Worten kann eine Biegeelastizität des Lagerungsschaftabschnitts durch die Schlitze oder die Wandstärke reduzierende Verjüngung erhöht/ eingestellt/ (vor-) bestimmt werden. Bei den Schlitzen kann es sich um ein lasergeschnittenes Muster in oder an dem Lagerungsschaft bzw. dem Lagerungsschaftabschnitt handeln. Bei der Verjüngung kann es sich um eine Reduzierung der Wandstärke des Lagerungsschafts, insbesondere außen umfänglich, handeln.

In einem weiteren Aspekt kann alternativ oder zusätzlich die Innenhülse oder Zwischenhülse die Schlitze oder die Wandstärke reduzierende Verjüngung beinhalten, um die Biegeelastizität einzustellen.

In einem weiteren Aspekt kann das lasergeschnittene Muster bzw. können die Schlitze bzw. die Verjüngung über eine Längserstreckung des Lagerungsschaftabschnitts bzw. der Innenhülse oder Zwischenhülse konstant sein. Alternativ kann das lasergeschnittene Muster bzw. können die Schlitze bzw. die Verjüngung sich über die Längserstreckung, beispielsweise in einer geometrischen Ausprägung, verändern.

In einem weiteren Aspekt kann der elastisch verformbare Lagerungsschaftabschnitt ein Kugelgelenk, vorzugsweise ein zweiachsiges Kugelgelenk, beinhalten.

In anderen Worten kann der Lagerungsschaft als zweiteiliger Lagerungsschaft ausgebildet sein, wobei ein erster Teil des Lagerungsschafts und ein zweiter Teil des Lagerungsschafts über das Kugelgelenk verbunden sein können. Der erste Teil des Lagerungsschafts kann mit einem Kugelkörper und der zweite Teil des Lagerungsschafts kann mit einem Schalenkörper ausgebildet sein. Der Kugelkörper kann in dem Schalenkörper gelagert sein.

In einem weiteren Aspekt kann das Kugelgelenk zwei Rotationsachsen beinhalten, wobei die zwei Rotationsachsen des zweiachsigen Kugelgelenks orthogonal zueinander angeordnet sind.

Der Kugelkörper kann in einem Aspekt zumindest einen Überstand, vorzugsweise in Form von einer Nase oder einem Pin, beinhalten, welcher in zumindest eine Nut oder eine Führung in dem Schalenkörper eingreift. Die Nut oder die Führung kann sich in Lagerungsschaftlängsrichtung erstrecken. Durch eine derartige Kombination von Überstand und Nut kann eine Rotation des ersten Teils des Lagerungsschafts in Relation zu dem zweiten Teil des Lagerungsschafts um eine Mittelfaser des Lagerungsschafts verhindert werden.

In einem weiteren Aspekt kann der Schalenkörper aus einzelnen Schalensegmenten ausgebildet sein, die sich (in einem montierten Zustand) tulpenförmig um den Kugelkörper legen und bei einer Montage federnd zurückweichen können.

In einem weiteren Aspekt kann die Erfassungsvorrichtung oder zumindest Teile der Erfassungsvorrichtung in dem Kugelgelenk ausgebildet sein. Konkret können in einem Hohlraum des Kugelgelenks bzw. an dem Kugelgelenk zumindest ein Dehnmessstreifen oder Force Sensitive Resistor ausgebildet sein. Der Force Sensitive Resistor kann normal/ quer zu der Längserstreckung des Lagerungsschafts ausgerichtet sein, wohingegen der Dehnmessstreifen parallel zu der Längserstreckung ausgerichtet sein kann.

In einem weiteren Aspekt kann das Kugelgelenk mit Druckfedern ausgebildet sein, die auf den Force Sensitive Resistor eine Druckkraft in Abhängigkeit des Biegeverformungszustands und/ oder der Biegeverformungsbewegung und/ oder des Grads der Biegeverformung ausüben.

In einem weiteren Aspekt kann benachbart zu dem Kugelgelenk eine Anlagefläche ausgebildet sein, den zumindest einen Force Sensitive Resistor aufzunehmen.

In einem weiteren Aspekt können zwischen der Druckfeder und dem Force Sensitive Resistor Scheiben bzw. zumindest eine Scheibe ausgebildet sein, die Druckkraft gleichmäßig auf den Force Sensitive Resistor zu verteilen.

In einem weiteren Aspekt können in dem Lagerungsschaft, bevorzugt in dem verformbaren Lagerungsschaftabschnitt, Klebenuten ausgebildet sein, die Erfassungsvorrichtung oder zumindest Teile der Erfassungsvorrichtung, insbesondere in Form des Sensors, zu fixieren.

In einem weiteren Aspekt kann die Erfassungsvorrichtung aus zumindest dem einen Sensor und zumindest einem Stab (oder Seil) bestehen, wobei ein Ende des Stabs oder Seils in dem verformbaren Lagerungsschaftabschnitt und das andere Ende einen einem proximalen Endabschnitt des Lagerungsschafts ausgebildet ist. Der Stab oder das Seil kann als ein Kraftübertragungselement fungieren, wodurch eine Positionierung des Sensors freier gestaltet sein kann.

In einem Aspekt kann für jeden Sensor ein Stab oder Seil ausgebildet sein.

Der Stab oder das Seil kann in zumindest einer (Längs-) Bohrung des Lagerungsschafts geführt sein.

Weiterhin wird die Aufgabe gelöst durch das medizinische Handstück zum rotatorischen und/ oder oszillierenden Antreiben des medizinischen Schaftwerkzeugs, mit der medizinischen Aufnahmevorrichtung nach einem der obigen Aspekte.

In anderen Worten kann das medizinische Handstück mit der medizinischen Aufnahmevorrichtung in oder an dem Lagerungsschaft ausgebildet sein.

Weiterhin wird die Aufgabe gelöst durch ein System aus dem medizinischen Handstück nach dem obigen Aspekt und einer Auswerteeinheit, wobei die Auswerteeinheit Signale der Erfassungsvorrichtung, welche einen Biegeverformungszustand und/ oder Biegeverformungsbewegung und/ oder einen Grad der Biegeverformung des Lagerungsschaftabschnitts des medizinischen Handstücks darstellen, auswertet, insbesondere mit hinterlegten Referenzdaten abgleicht, und, basierend auf der Auswertung, einen Verschleißgrad eines medizinischen Schaftwerkzeugs bestimmt.

In anderen Worten beinhaltet das System die Auswerteeinheit, die separat von dem medizinischen Handstück, beispielsweise in einer Steuerungsvorrichtung des medizinischen Handstücks, welche über ein Kabel oder drahtlos mit dem medizinischen Handstück verbunden sein kann, ausgebildet ist. Die Auswerteeinheit erhält die Signale der Erfassungsvorrichtung, insbesondere über die Leiterbahnen, die (unter anderem) in dem Lagerungsschaft ausgebildet sein können. Die Auswerteeinheit wertet die erhaltenen Signale aus und bestimmt basierend auf der Auswertung den Verschleißgrad des medizinischen Schaftwerkzeugs. Die Auswerteeinheit kann die Auswertung basierend auf einem Abgleich der Signale mit den hinterlegten Referenzdaten durchführen.

In einem Aspekt können die hinterlegten Referenzdaten mit einer künstlichen Intelligenz trainiert sein bzw. trainiert werden.

In einem weiteren Aspekt kann die Auswerteeinheit mit einer Datenbank, vorzugsweise einer Cloud, verbunden sein und Referenzdaten aus der Datenbank beziehen bzw. auf die Referenzdaten der Datenbank zugreifen.

In einem weiteren Aspekt kann die Auswerteeinheit eine Eingabeschnittstelle beinhalten, welche eine Eingabe des (spezifischen) Schaftwerkzeugs und/ oder Kenndaten des (spezifischen) Schaftwerkzeugs in die Auswerteeinheit ermöglicht. Bei der Eingabeschnittstelle kann es sich um eine manuelle Eingabeschnittstelle, wie beispielsweise ein Touchscreen oder eine Tastatur, handeln.

Alternativ oder zusätzlich kann die Eingabeschnittstelle mit einer Ausleseeinheit ausgebildet sein, welche ein drahtloses Erkennungselement des Schaftwerkzeugs, beispielsweise ein RFID-Tag, ausliest und das Schaftwerkzeug automatisiert erkennt.

In einem weiteren Aspekt kann die Auswerteeinheit individuelle Verschleißwerte für individuelle Schaftwerkzeuge in der Datenbank hinterlegen.

In einem weiteren Aspekt kann die Auswerteeinheit eine Lebensdauerprädiktionseinheit beinhalten, welche basierend auf dem ermittelten Verschleißgrad eine Lebensdauerprädiktion und / oder eine Austauschempfehlung für das medizinische Schaftwerkzeug ausgibt.

In anderen Worten kann die Auswerteeinheit die Lebensdauerprädiktionseinheit beinhalten. Die Lebensdauerprädiktionseinheit kann mit der künstlichen Intelligenz trainiert sein. Alternativ oder zusätzlich kann die Lebensdauerprädiktionseinheit mit Referenzdaten trainiert sein. Die Lebensdauerprädiktionseinheit kann durch einen Abgleich des ermitteln Verschleißgrads mit hinterlegten Daten vorhersagen, wann ein Austausch des Schaftwerkzeugs voraussichtlich notwendig sein wird. Alternativ oder zusätzlich kann die Lebensdauerprädiktionseinheit eine Austauschempfehlung für das medizinische Schaftwerkzeug ausgeben, wenn die Lebensdauerprädiktionseinheit bestimmt, dass ein maximal zulässiger Verschleißgrad erreicht ist. Der maximal zulässige Verschleißgrad kann durch den Bediener eingegeben oder durch die künstliche Intelligenz ermittelt werden.

In einem weiteren Aspekt kann die Auswerteeinheit Energieströme, beispielsweise des medizinischen Handstücks oder des medizinischen Roboters, berücksichtigen. In anderen Worten können Energieströme wie beispielsweise Antriebsströme von der Auswerteeinheit zur Ermittlung des Verschleißgrads hinzugezogen werden.

In einem weiteren Aspekt kann die Auswerteeinheit Temperaturfühler, Dehnmessstreifen und/oder Beschleunigungssensoren am medizinischen Handstück oder am Eingriffsort beinhalten.

In einem weiteren Aspekt kann das System zusätzliche Erkennungskomponenten beinhalten, sodass ein Verschleißgrad noch genauer erkannt werden kann. Mögliche zusätzliche Erkennungskomponenten sind eine High-Speed Kamera, eine Wärmebildkamera, Infrarottemperatursensoriken und dergleichen.

In einem weiteren Aspekt können Oberflächenergebnisse am Eingriffsort, am Schaftwerkzeug und an einem etwaigen Span über von den Kameras erfasste Bilddaten ausgewertet werden.

Die Aufgabe wird weiterhin gelöst durch ein Verfahren zum Ermitteln eines Verschleißgrads des medizinischen Schaftwerkzeugs mit den Schritten:
- Ermitteln eines Biegeverformungszustands und/oder einer Biegeverformungsbewegung und/ oder eines Grads der Biegeverformung eines Lagerungsschaftabschnitts eines Lagerungsschafts;
- Vergleich des Biegeverformungszustands und/oder der Biegeverformungsbewegung und/ oder des Grads der Biegeverformung des Lagerungsschaftabschnitts mit Referenzdaten;
- Bestimmen eines Verschleißgrades basierend auf dem Vergleich;
- Ausgabe des Verschleißgrades.

In einem Aspekt kann das Verfahren weiterhin den Schritt beinhalten:
- Ausgabe einer Lebensdauerprädiktion und/ oder einer Austauschempfehlung für das medizinische Schaftwerkzeug.

In einem weiteren Aspekt kann in dem Schritt des Vergleichens eine Vorschubgeschwindigkeit des Werkzeugs berücksichtigt werden.

In einem weiteren Aspekt kann in dem Schritt des Vergleichens eine Drehzahl und/ oder ein Winkel/ ein Eingriffswinkel des Schaftwerkzeugs berücksichtigt werden.

In einem weiteren Aspekt kann das Verfahren weiterhin den Schritt beinhalten:
- Anpassen von Antriebsparametern des medizinischen Schaftwerkzeugs basierend auf dem bestimmten Verschleißgrad des medizinischen Schaftwerkzeugs.

In anderen Worten können die Antriebsparameter, wie beispielsweise Vorschub und Drehzahl des Schaftwerkzeugs an die aktuell auftretende äußere Belastung angepasst werden.

In einem weiteren Aspekt kann in Kombination mit der künstlichen Intelligenz eine Verfahrbewegung und/ oder eine Arbeitsroutine, insbesondere in Kombination mit dem medizinischen/ chirurgischen Roboter, auf Basis der erfassten Signale bzw. der erkannten äußeren Belastung angepasst werden.

### Kurzbeschreibung der Figuren

Fig. 1 ist eine Darstellung eines offenbarungsgemäßen medizinischen Handstücks gemäß einer ersten Ausführungsform;
Fig. 2 ist eine Schnittdarstellung des offenbarungsgemäßen medizinischen Handstücks gemäß der ersten Ausführungsform mit einer ersten Detailansicht;
Fig. 3 ist eine Darstellung des offenbarungsgemäßen medizinischen Handstücks gemäß einer zweiten Ausführungsform;
Fig. 4 ist eine Schnittdarstellung des offenbarungsgemäßen medizinischen Handstücks gemäß der zweiten Ausführungsform mit einer zweiten Detailansicht;
Fig. 5 ist eine Schnittdarstellung des offenbarungsgemäßen medizinischen Handstücks gemäß einer dritten Ausführungsform mit einer dritten Detailansicht und einer vierten Detailansicht;
Fig. 6 ist eine Darstellung des offenbarungsgemäßen medizinischen Handstücks gemäß einer vierten Ausführungsform;
Fig. 7 ist eine Schnittdarstellung des offenbarungsgemäßen medizinischen Handstücks gemäß der vierten Ausführungsform mit einer fünften Detailansicht;
Fig. 8 ist eine Darstellung des offenbarungsgemäßen medizinischen Handstücks gemäß einer fünften Ausführungsform;
Fig. 9 ist eine Schnittdarstellung des offenbarungsgemäßen medizinischen Handstücks gemäß der fünften Ausführungsform mit einer sechsten Detailansicht und einer siebten Detailansicht;
Fig. 10 ist eine Darstellung des offenbarungsgemäßen medizinischen Handstücks gemäß einer sechsten Ausführungsform;
Fig. 11 ist eine Schnittdarstellung des offenbarungsgemäßen medizinischen Handstücks gemäß der sechsten Ausführungsform mit einer achten Detailansicht und einer neunten Detailansicht;
Fig. 12 ist eine Darstellung des offenbarungsgemäßen medizinischen Handstücks gemäß einer siebten Ausführungsform; und
Fig. 13 ist eine Schnittdarstellung des offenbarungsgemäßen medizinischen Handstücks gemäß der siebten Ausführungsform mit einer zehnten Detailansicht, einer elften Detailansicht und einer zwölften Detailansicht.

### Beschreibung der Ausführungsformen

Nachstehend werden Ausführungsformen der vorliegenden Offenbarung auf der Basis der zugehörigen Figuren beschrieben.

### Erste Ausführungsform

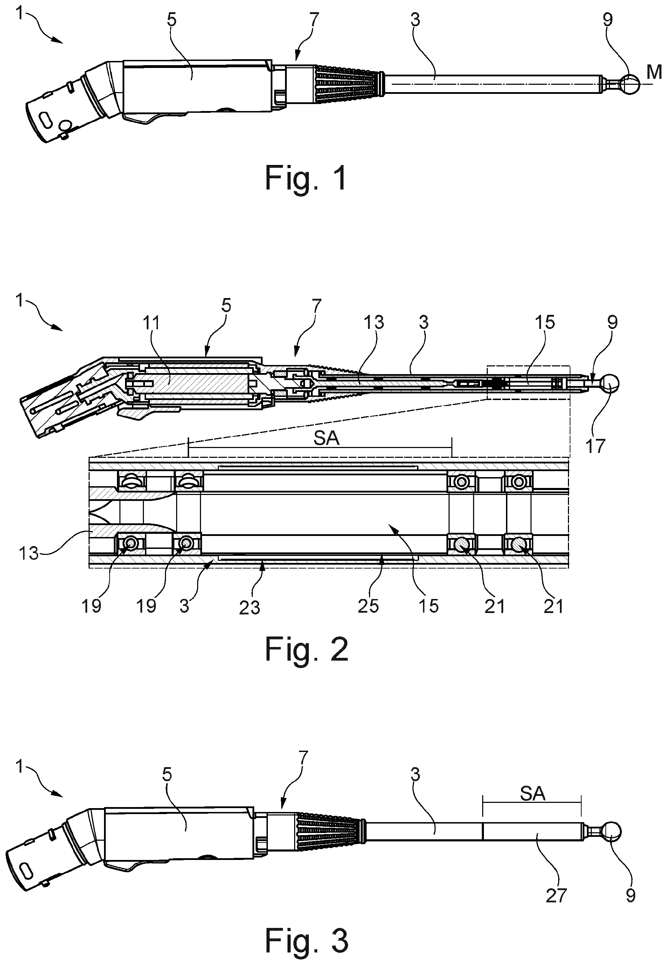

Fig. 1 zeigt ein offenbarungsgemäßes medizinisches Handstück 1 gemäß einer ersten Ausführungsform mit einem Lagerungsschaft 3, welcher lösbar mit einem Griffabschnitt 5 über eine Kupplung 7 verbunden ist. Der Lagerungsschaft 3 weist im Wesentlichen eine rohrförmige Geometrie auf. An einem distalen Ende des medizinischen Handstücks 1 bzw. des Lagerungsschafts 3 ist eine Aufnahme für ein medizinisches Schaftwerkzeug 9 ausgebildet. Der Lagerungsschaft 3 ist vorgesehen und ausgebildet, eine von dem Griffabschnitt 5 erzeugte rotatorische oder oszillierende Antriebskraft auf das medizinische Schaftwerkzeug 9 zu übertragen. Die Antriebskraft kann beispielsweise durch einen elektrischen, einen hydraulischen oder einen pneumatischen Antrieb 11 (siehe Fig. 2) in dem Griffabschnitt 5 erzeugt werden.

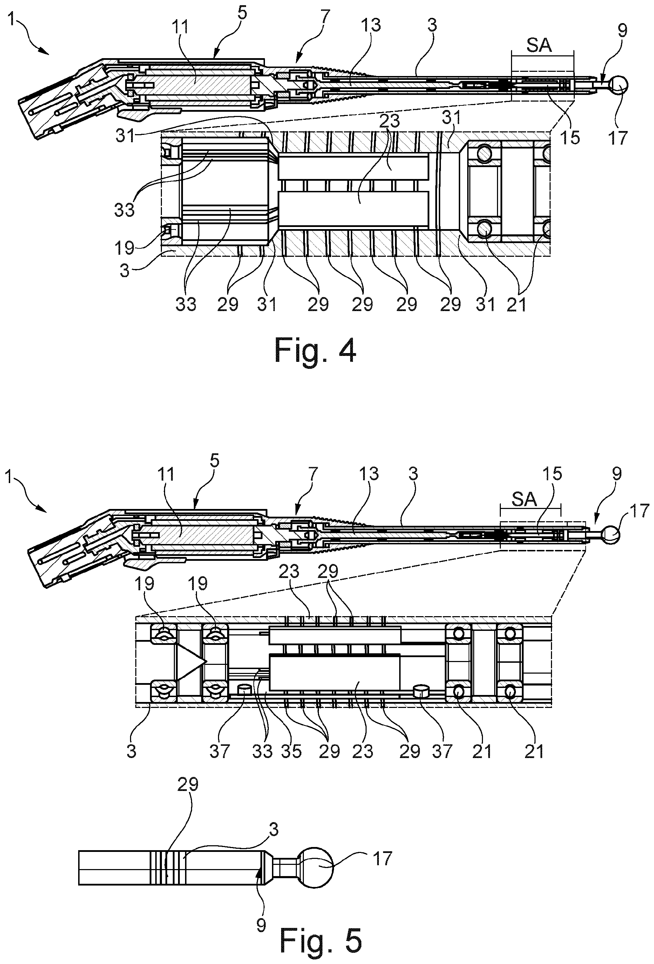

Fig. 2 zeigt eine Schnittansicht des medizinischen Handstücks 1 aus Fig. 1. In dem Lagerungsschaft 3 ist eine Antriebswelle 13 drehbar gelagert, die von dem Antrieb 11 erzeugte Antriebskraft auf das Schaftwerkzeug 9 zu übertragen. Das Schaftwerkzeug 9 beinhaltet einen Werkzeugschaft 15 und einen Effektor 17. Bei dem Effektor 17 kann es sich beispielsweise um einen Fräskopf, einen Bohrer oder dergleichen handeln. Der Werkzeugschaft 15 und die Antriebswelle 13 sind nach einem "Plug and Play System" miteinander verbunden/ verbindbar. Der Werkzeugschaft 15 ist drehbar in dem Lagerungsschaft 3 gelagert. Konkret ist der Werkzeugschaft 15 in einer ersten Lagerstelle 19 und einer zweiten Lagerstelle 21 gelagert. Die erste Lagerstelle 19 und die zweite Lagerstelle 21 sind in der gezeigten Ausführungsform als Kugellager bzw. Kugellagerpaare ausgebildet. Selbstverständlich sind auch andere geeignete Lager anstelle der Kugellager vorstellbar. Die erste Lagerstelle 19 und die zweite Lagerstelle 21 sind beabstandet zueinander in dem Lagerungsschaft 3 ausgebildet. An einer Innenfläche des Lagerungsschafts 3 ist ein Sensor in Form eines Dehnmessstreifens 23 ausgebildet. Der Dehnmessstreifen 23 wird in einer Richtung hin zu dem Werkzeugschaft 15 durch eine Zwischenhülse 25 begrenzt. Die Zwischenhülse 25 positioniert die Lager der ersten Lagerstelle 19 und die Lager der zweiten Lagerstelle 21 in einer Längsrichtung des Lagerungsschafts 3 zueinander. Weiterhin kann die Zwischenhülse 25 mit Leiterbahnen (siehe 33 Fig. 4) ausgebildet sein, welche ein von dem Dehnmessstreifen 23 erzeugtes Messsignal an ein proximales Ende des Lagerungsschafts 3 bzw. des medizinischen Handstücks 1 weiterleiten können. Neben der Zwischenhülse sind auch die Lager der ersten Lagerstelle 19 und alle weiteren Zwischenhülsen und Lagerstellen, beispielsweise die zweite Lagerstelle 21, in Richtung proximal mit Leiterbahnen 33 ausgebildet, um eine Messsignalleitung zur Auswerteeinheit 69 zu ermöglichen.

Der Lagerungsschaft 3 ist in der ersten Ausführungsform aus einem (federnd) elastischen Material ausgebildet. In anderen Worten ist der Lagerungsschaft 3 als solches (federnd) beweglich bzw. elastisch biegsam ausgebildet und durch eine normal zu einer Mittelfaser M des Lagerungsschafts 3 wirkende Kraft/ Radialkraft elastisch auslenkbar. Der Dehnmessstreifen 23 ist vorgesehen und ausgebildet, eine elastische Auslenkung bzw. die Biegung des Lagerungsschafts 3, insbesondere in einem Lagerungsschaftabschnitt SA zwischen der ersten Lagerstelle 19 und der zweiten Lagerstelle 21, welche durch eine radial auf das in dem Lagerungsschaft 3 positionierte medizinische Werkzeug 3 wirkende Kraft und/ oder äußere Belastung erzeugt wird, zu detektieren.

### Zweite Ausführungsform

Fig. 3 und Fig. 4 zeigen das offenbarungsgemäße medizinische Handstück 1 gemäß einer zweiten Ausführungsform. Nachfolgend wird lediglich auf Unterschiede zu der anhand von Fig. 1 und Fig. 2 beschriebenen ersten Ausführungsform eingegangen.

In dem Lagerungsschaftabschnitt SA der zweiten Ausführungsform ist der Lagerungsschaft 3 außen umfänglich mit einer Schutzschicht/ Schutzhülle/ Ummantelung, beispielsweise einem Schrumpfschlauch 27, vor Umwelteinflüssen, wie beispielsweise Schmutzpartikeln und/oder flüssige Medien, geschützt. Die Schutzschicht kann auch verwendet werden, die Biegeelastizität des Lagerungsschaftabschnitts einzustellen.

Fig. 4 zeigt eine Schnittansicht des medizinischen Handstücks 1 aus Fig. 3 mit einer Detailansicht des Schaftabschnitts SA, wobei in der Detailansicht zur besseren Veranschaulichung das Schaftwerkzeug 9 nicht dargestellt ist. Der Lagerungsschaft 3 ist in dem Lagerungsschaftabschnitt SA mit einem lasergeschnittenen Muster in Form von Schlitzen 29 ausgebildet, um die Elastizität des Lagerungsschafts 3 zu erhöhen und die Biegeelastizität auf einen gewünschten bzw. vorbestimmten Wert einzustellen.

In der zweiten Ausführungsform ist weiterhin auf eine Zwischenhülse 25 verzichtet. In dem Lagerungsschaft 3 sind in der zweiten Ausführungsform hingegen Schultern 31 ausgebildet, welche die Lager der ersten Lagerstelle 19 und der zweiten Lagerstelle 21 positionieren. Die Schultern sind einstückig, insbesondere stoffeinstückig, mit dem Lagerungsschaft 3 ausgebildet. Die Dehnungsmesstreifen 23 sind zwischen der ersten Lagerstelle 19 und der zweiten Lagerstelle 21 an der Innenfläche des Lagerungsschafts 3 ausgebildet/ angebracht. Die Dehnungsmesstreifen 23 sind über die Leiterbahnen 33 kontaktierbar/ kontaktiert. Die Dehnungsmesstreifen 23 sind beispielsweise durch kleben an der Innenfläche des Lagerungsschafts 3 fixiert. Optional sind die Schlitze 29 mit Elastomermodulen gefüllt oder mit einem Elastomer vergossen, um eine bessere Reinigbarkeit des Schafts zu ermöglichen.

### Dritte Ausführungsform

Fig. 5 zeigt eine Schnittansicht des medizinischen Handstücks 1 gemäß einer dritten Ausführungsform. Die dritte Ausführungsform entspricht im Wesentlichen der zweiten Ausführungsform, sodass nachfolgend lediglich auf Unterschiede der dritten Ausführungsform zu der zweiten Ausführungsform eingegangen wird.

In der dritten Ausführungsform sind keine Schultern 31 in dem Lagerungsschaft 3 ausgebildet. Anstelle der Schultern ist eine geschlitzte Innenhülse 35 zwischen der ersten Lagerstelle 19 und der zweiten Lagerstelle 21 ausgebildet. Die Dehnmessstreifen 23 sind an einer Innenfläche der geschlitzten Innenhülse 35 ausgebildet bzw. fixiert. Weiterhin sind die Leiterbahnen 33 in der geschlitzten Innenhülse 35 ausgebildet. Die geschlitzte Innenhülse 35 positioniert die erste Lagerstelle 19 und die zweite Lagerstelle 21 zueinander. Für eine eindeutige Positionierung zwischen dem Lagerungsschaft 3 und der geschlitzten Innenhülse 35 sorgen zwei Positionierungsstifte 37, welche in entsprechende Öffnungen des Lagerungsschafts 3 und der geschlitzten Innenhülse 35 eingreifen und die geschlitzte Innenhülse 35 und den Lagerungsschaft 3 zumindest formschlüssig miteinander verbinden. Die Positionierungsstifte 37 können in den Lagerungsschaft 3 eingepresst, eingeklebt oder anderweitig geeignet fixiert werden.

### Vierte Ausführungsform

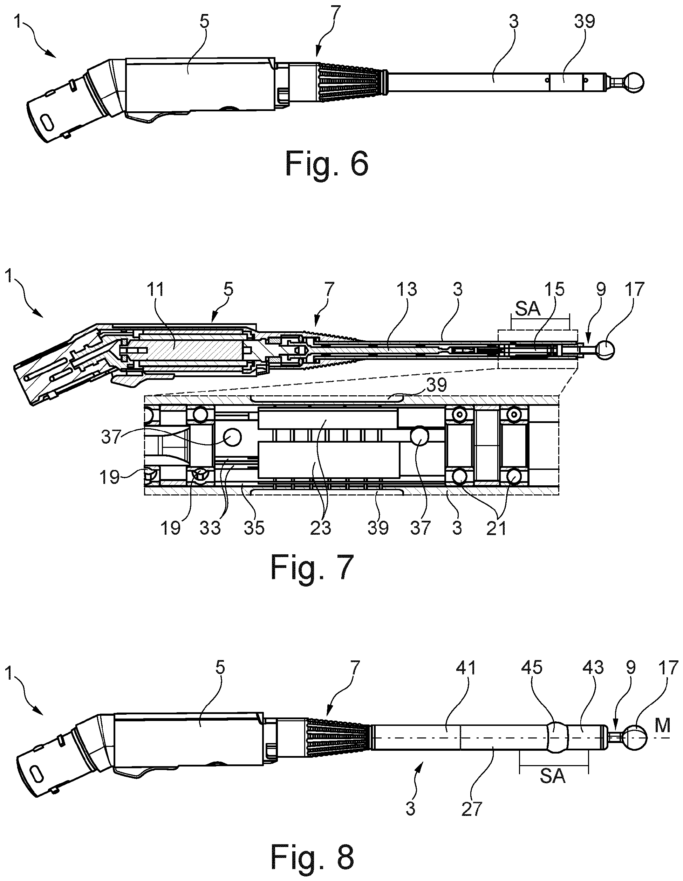

Fig. 6 und Fig. 7 zeigen das medizinische Handstück 1 gemäß einer vierten Ausführungsform. Die vierte Ausführungsform entspricht im Wesentlichen der dritten Ausführungsform, sodass nachfolgend lediglich auf Unterschiede der vierten Ausführungsform zu der dritten Ausführungsform eingegangen wird.

In der vierten Ausführungsform sind keine Schlitze 29 in dem Lagerungsschaft 3 ausgebildet. Hingegen wird eine Beweglichkeit des Lagerungsschafts 3 durch eine Verjüngung 39 des Schaftdurchmessers innerhalb des Lagerungsschaftabschnitts SA erzielt. Die Verjüngung 39 kann dabei beispielsweise durch eine Wandstärkenreduktion um ca. 50 % ausgebildet sein. Selbstverständlich sind auch andere Werte für die Wandstärkenreduktion möglich. Die Verjüngung 39 kann optional mit einem Elastomer vergossen sein, um Kanten in dem Lagerungsschaft 3 zu verhindern und die Reinigbarkeit zu verbessern. In der hier dargestellten vierten Ausführungsform ist die Verjüngung 39 durch eine umfängliche Ausnehmung in einer Schaftaußenseite des Lagerungsschafts 3 umgesetzt. Alternativ ist auch vorstellbar, die umfängliche Ausnehmung in der Innenseite/ Innenfläche des Lagerungsschafts 3 auszubilden.

In der hier dargestellten vierten Ausführungsform ist die geschlitzte Innenhülse 35 ausgebildet. Alternativ kann eine Innenhülse/ Zwischenhülse ausgebildet sein, welche ebenfalls durch eine dünnere Wandstärke in ihrer Beweglichkeit erhöht werden kann. Weiterhin alternativ kann die vierte Ausführungsform auch komplett ohne eine Innenhülse mit Schultern 31 wie in der zweiten Ausführungsform ausgebildet sein.

Während die ersten vier Ausführungsformen mit einem einteiligen Lagerungsschaft 3 ausgebildet sind, welcher allein aufgrund seiner Elastizität bzw. der Elastizität des Materials des Lagerungsschafts 3 gebogen werden kann, werden in den nachfolgenden Ausführungsformen Varianten beschriebenen, bei denen mittels eines Kugelgelenks eine notwendige Beweglichkeit ermöglicht wird. Anders ausgedrückt ist in den nachfolgenden Ausführungsformen der Lagerungsschaft 3 zweiteilig.

### Fünfte Ausführungsform

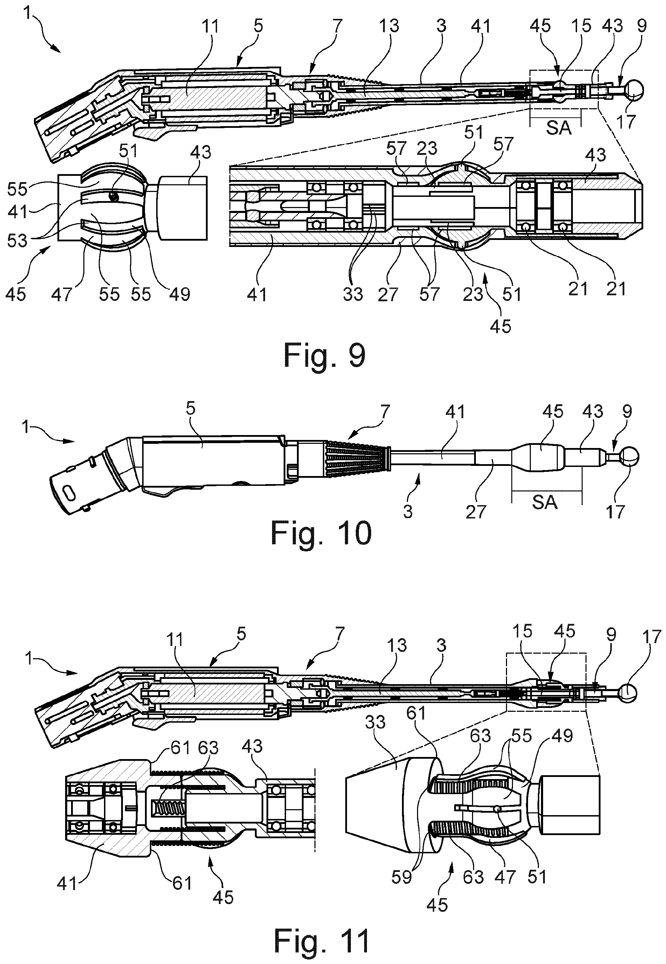

Fig. 8 und Fig. 9 zeigen das offenbarungsgemäße medizinische Handstück 1 gemäß einer fünften Ausführungsform.

Der Lagerungsschaft 3 beinhaltet einen ersten proximalen Schaftteil 41 und einen zweiten distalen Schaftteil 43 welche über ein Kugelgelenk 45 beweglich miteinander verbunden sind. Das Kugelgelenk 45 ist in dem Lagerungsschaftabschnitt SA ausgebildet. Das Kugelgelenk 45 beinhaltet einen tulpenförmigen Schalenabschnitt 47, der an dem ersten proximalen Schaftteil 41 ausgebildet ist, und einem Kugelabschnitt 49, der an dem zweiten distalen Schaftteil 43 ausgebildet ist. Der Kugelabschnitt 49 ist in dem Schalenabschnitt 47 beweglich gelagert. Eine Rotation des Kugelabschnitts 49 gegenüber des Schalenabschnitts 47 um die Mittelfaser M ist durch Zapfen 51, die an dem Kugelabschnitt 49 ausgebildet sind und in Ausnehmungen 53 des Schalenabschnitts 47 gleiten, verhindert. Die Ausnehmungen 53 erstrecken sich im Wesentlichen in der Richtung der Mittelfaser M bzw. parallel zu der Mittelfaser M, sodass eine Kippbewegung zwischen dem ersten proximalen Schaftteil 41 und dem zweiten distalen Schaftteil 43 ermöglicht ist. Das Kugelgelenk 45 ist umfänglich durch den Schrumpfschlauch 27 geschützt. Einzelne Segmente 55 des Schalenabschnitts 47 sind elastisch ausgebildet, sodass bei einer Montage der Kugelabschnitt 49 in den Schalenabschnitt 47 eingeführt werden kann und der Schalenabschnitt 47 zurück in seine Ausgangsposition/ Ausgangsgeometrie federt.

In der fünften Ausführungsform sind die Dehnmessstreifen 23 in dem Kugelgelenk 45 ausgebildet und an gegenüberliegenden Enden jeweils mit dem ersten proximalen Schaftteil 41 und dem zweiten distalen Schaftteil 43 verbunden. Zur Fixierung der Dehnmessstreifen 23 sind sowohl in dem ersten proximalen Schaftteil 41 und dem zweiten distalen Schaftteil 43 Klebstoffnuten 57 ausgebildet, welche mit Klebstoff gefüllt werden können, um die Dehnmessstreifen 23 zu fixieren.

Analog zu den vorherigen Ausführungsformen erfolgt eine Signalleitung von dem Dehnmessstreifen 23 auf einen hinteren Teil des Handstücks über die Leiterbahnen 33, die in Hülsen und Kugellagern ausgebildet sind.

### Sechste Ausführungsform

Fig. 10 und Fig. 11 zeigen das medizinische Handstück gemäß einer sechsten Ausführungsform. Die sechste Ausführungsform entspricht im Wesentlichen der fünften Ausführungsform, sodass nachfolgend lediglich auf Unterschiede der sechsten Ausführungsform zu der fünften Ausführungsform eingegangen wird.

In der sechsten Ausführungsform werden anstelle der Dehnmessstreifen 23 Force Sensitive Resistors 59 verwendet. Die Force Sensitive Resistors 59 sind senkrecht zu der Mittelachse M an einer planen Fläche 61 des ersten proximalen Schaftteils 41 ausgebildet. Analog zu der Dehnungsänderung, die bei den Dehnmessstreifen 23 gemessen wird, ändert sich bei den Force Sensitive Resistors 59 ein (elektrischer) Widerstand durch eine senkrecht aufgebrachte Druckkraft. Diese Druckkraft wird in der sechsten Ausführungsform durch den relativ zu dem ersten proximalen Schaftteil 41 über das Kugelgelenk 45 beweglichen zweiten distalen Schaftteil 43 über geführte Druckfedern 63 und Scheiben (nicht dargestellt) auf den jeweiligen Force Sensitive Resistor 59 übertragen.

### Siebte Ausführungsform

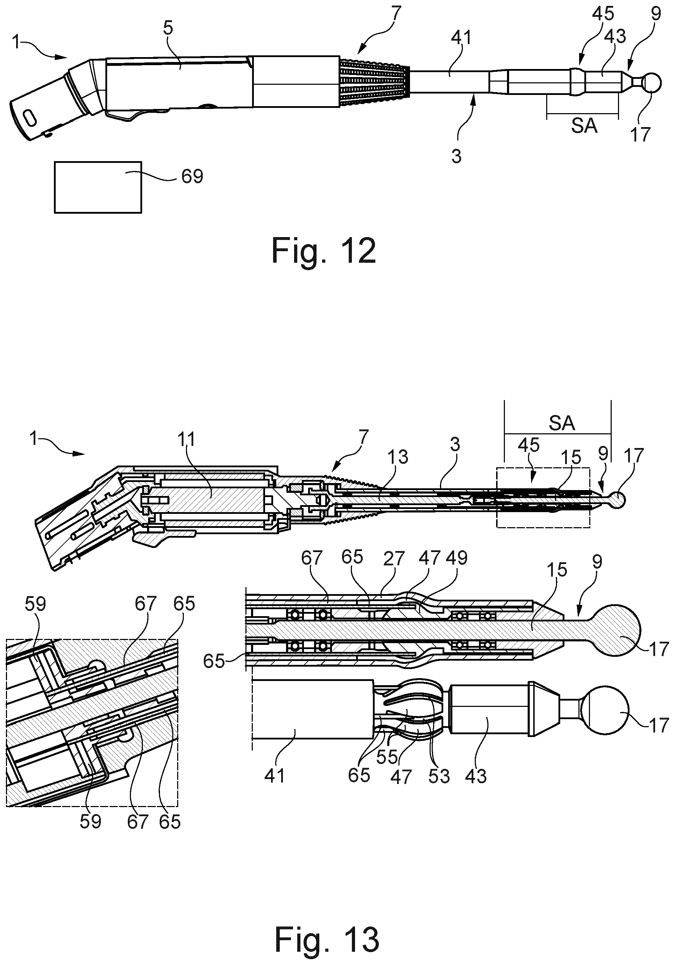

Fig. 12 und Fig. 13 zeigen das medizinische Handstück gemäß einer siebten Ausführungsform. Die siebte Ausführungsform entspricht im Wesentlichen der sechsten Ausführungsform, sodass nachfolgend lediglich auf Unterschiede der siebten Ausführungsform zu der sechsten Ausführungsform eingegangen wird.

Im Unterschied zu der sechsten Ausführungsform wird die Druckkraft bzw. Zugkraft nicht über die Druckfedern 63 auf den jeweiligen Force Sensitive Resistor 59 übertragen, sondern über Stäbe 65 (alternativ auch über Seile bzw. Drahtseile). Die Force Sensitive Resistors 59 befinden sich in der siebten Ausführungsform am distalen Ende des Lagerungsschafts 3 bzw. alternativ in dem Griffabschnitt 5 des medizinischen Handstücks 1. Die Stäbe 65 werden durch Bohrungen 67 in dem ersten proximalen Schaftteil 41 geführt.

In Fig. 12 ist weiterhin eine Auswerteeinrichtung/ Auswerteeinheit 69 schematisch dargestellt. Die Auswerteeinrichtung beinhaltet optional zusätzlich eine Lebensdauerprädiktionseinheit und/ oder eine Eingabevorrichtung.

Selbstverständlich sind die beschriebenen Merkmalskombinationen in den Ausführungsformen nicht abschließend zu verstehen. Viel mehr können Merkmale, wenn dies technisch sinnvoll möglich ist, zwischen den unterschiedlichen Ausführungsformen ausgetauscht werden.

Nachfolgend wird eine allgemeine beispielhafte Funktionsweise der offenbarungsgemäßen medizinischen Aufnahmevorrichtung bzw. des offenbarungsgemäßen Systems anhand der Figuren erläutert.

Bei dem chirurgischen Behandlungsvorgang wird durch einen Behandler eine Kraft auf das medizinische Handstück 1 ausgeübt. Diese Kraft ist abhängig von einer Schnittleistung des Schaftwerkzeugs 9 und ändert sich über einen Behandlungsfortschritt in Abhängigkeit von dem Verschleiß des Schaftwerkzeugs 9. Die ausgeübte Kraft führt zu einer Biegeverformung des Lagerungsschaftabschnitts SA. Die Biegeverformung des Lagerungsschaftabschnitts SA wird von dem Sensor (dem Dehnmessstreifen 23 oder dem Force Sensitive Resistor 59) in ein Signal übersetzt. Das Signal wird über die Leiterbahnen 33 zu einem proximalen Ende des medizinischen Handstücks geleitet. Eine Auswerteeinheit 69 wertet die Signale aus und gibt, basierend auf der Auswertung einen Verschleißgrad des Schaftwerkzeugs 9 und/ oder eine Lebensdauerprädiktion für das Schaftwerkzeug 9 und oder eine Austauschempfehlung für das Schaftwerkzeug 9 aus.
In einer alternativen Ausführungsform können mehrere Dehnungsmessstreifen 23 oder Force Sensitive Resistors 59 in radialer Richtung um den Umfang verteilt angebracht sein, um so Kräfte in unterschiedlichen Achsen gleichzeitig zu erfassen bzw. auszuwerten. Dadurch können Kräfte aus allen Richtungen ausgewertet werden, was besonders bei handgehaltener Anwendung wichtig ist, da hier die resultierende Bewegung des Handstücks nicht bekannt oder vorherbestimmbar ist, wie es beispielsweise bei einer robotischen Anwendung der Fall ist.

### Bezugszeichenliste

- 1: medizinisches Handstück
- 3: Lagerungsschaft/ Schaft
- 5: Griffabschnitt
- 7: Kupplung
- 9: medizinisches Schaftwerkzeug! Werkzeug
- 11: Antrieb
- 13: Antriebswelle
- 15: Werkzeugschaft
- 17: Effektor
- 19: erste Lagerstelle
- 21: zweite Lagerstelle
- 23: Dehnmessstreifen
- 25: Zwischenhülse
- 27: Schrumpfschlauch
- 29: Schlitz
- 31: Schulter
- 33: Leiterbahn
- 35: geschlitzte Innenhülse
- 37: Positionierungsstift/ Stift
- 39: Verjüngung
- 41: proximaler Schaftteil
- 43: distaler Schaftteil
- 45: Kugelgelenk
- 47: Schalenabschnitt
- 49: Kugelabschnitt
- 51: Zapfen
- 53: Ausnehmung
- 55: Segment
- 57: Klebstoffnut
- 59: Force Sensitive Resistor/ FSR/ Force Sensing Resistor
- 61: (plane) Fläche
- 63: Druckfeder
- 65: Stab
- 67: Bohrung
- 69: Auswerteeinheit/ Auswerteeinrichtung
- M: Mittelfaser
- SA: Lagerungsschaftabschnitt

## Patentansprüche

1. Medizinische Detektions- oder Aufnahmevorrichtung zur Detektion oder Aufnahme einer äußeren Belastung eines chirurgischen Schaftwerkzeugs (9) während eines chirurgischen Behandlungsvorgangs, aufweisend:
- einen Lagerungsschaft (3) für eine oder einer chirurgischen Behandlungsvorrichtung (1), der zur lagernden Aufnahme und/ oder Abstützung des chirurgischen Schaftwerkzeugs (9) ausgebildet ist,
- zumindest einen elastisch verformbaren Lagerungsschaftabschnitt (SA), der eine bestimmte, vorzugsweise vorbestimmte Biegeelastizität hat und
- eine Erfassungsvorrichtung oder Sensorik, die dafür ausgebildet ist, einen Biegeverformungszustand und/ oder Biegeverformungsbewegung und/ oder einen Grad der Biegeverformung des Lagerungsschaftabschnitts (SA) infolge der äußeren Belastung zu erfassen.

2. Medizinische Aufnahmevorrichtung nach Anspruch 1, wobei die Erfassungsvorrichtung zumindest einen Sensor (23; 59), vorzugsweise ein Kraftsensor, insbesondere ein Piezosensor oder ein Force Sensing Resistor, beinhaltet.

3. Medizinische Aufnahmevorrichtung nach Anspruch 2, wobei die Erfassungsvorrichtung zumindest zwei Sensoren (23, 59) aufweist, welche orthogonal zueinander in dem Lagerungsschaft (3) angeordnet sind.

4. Medizinische Aufnahmevorrichtung nach einem der Ansprüche 1 bis 3, wobei der Lagerungsschaft (3) im Lagerungsschaftlängsabstand zueinander angeordnete Lagerstellen (19; 21) zur Lagerung oder Abstützung des medizinischen Schaftwerkzeugs (9) beinhaltet und die Erfassungsvorrichtung, zumindest teilweise, zwischen den Lagerstellen (19; 21) angeordnet ist.

5. Medizinische Aufnahmevorrichtung nach einem der Ansprüche 1 bis 4, wobei der Lagerungsschaft (3) einen proximalen Kopplungsabschnitt (7) zur Ankopplung des Lagerungsschafts (3) an die medizinische Behandlungsvorrichtung, vorzugsweise Handstück (5) und/ oder medizinischer Roboter beinhaltet, wobei elektrische Leiterbahnen (33), welche vorgesehen und ausgebildet sind, Signale der Erfassungsvorrichtung zu leiten, zwischen der Erfassungsvorrichtung und dem Kopplungsabschnitt (7) ausgebildet oder verlegt sind.

6. Medizinische Aufnahmevorrichtung nach einem der Ansprüche 1 bis 5, wobei in dem elastisch verformbaren Lagerungsschaftabschnitt (SA) eine Innenhülse (25; 35) ausgebildet ist, welche die zumindest eine Erfassungsvorrichtung beinhaltet.

7. Medizinische Aufnahmevorrichtung nach einem der Ansprüche 1 bis 6, wobei der elastisch verformbare Lagerungsschaftabschnitt (SA) Ausnehmungen (29; 39), vorzugsweise in Form von Schlitzen oder einer Wandstärke reduzierenden Verjüngung, beinhaltet.

8. Medizinische Aufnahmevorrichtung nach einem der Ansprüche 1 bis 6, wobei der elastisch verformbare Lagerungsschaftabschnitt (SA) ein Kugelgelenk (45), vorzugsweise ein zweiachsiges Kugelgelenk, beinhaltet.

9. Medizinische Aufnahmevorrichtung nach Anspruch 8, wobei das Kugelgelenk (45) zwei Rotationachsen beinhaltet und die beiden Rotationsachsen des zweiachsigen Kugelgelenks (45) orthogonal zueinander angeordnet sind.

10. Medizinisches Handstück (1) zum rotatorischen und/ oder oszillierenden Antreiben eines medizinischen Schaftwerkzeugs (9), mit einer medizinischen Aufnahmevorrichtung nach einem der Ansprüche 1 bis 9.

11. System aus einem medizinischen Handstück (1) nach Anspruch 10 und einer Auswerteeinheit (69), wobei die Auswerteeinheit (69) Signale einer Erfassungsvorrichtung, welche einen Biegeverformungszustand und/ oder Biegeverformungsbewegung und/ oder einen Grad der Biegeverformung des Lagerungsschaftabschnitts (SA) des medizinischen Handstücks (1) darstellen, auswertet, insbesondere mit hinterlegten Referenzdaten abgleicht, und basierend auf der Auswertung einen Verschleißgrad eines medizinischen Schaftwerkzeugs (9) bestimmt.

12. System nach Anspruch 11, wobei die Auswerteeinheit (69) eine Lebensdauerprädiktionseinheit beinhaltet, welche basierend auf dem ermittelten Verschleißgrad eine Lebensdauerprädiktion und/ oder eine Austauschempfehlung für das medizinische Schaftwerkzeug (9) ausgibt.

13. Verfahren zum Ermitteln eines Verschleißgrads eines medizinischen Schaftwerkzeugs (9) mit den Schritten:
- Ermitteln eines Biegeverformungszustands und/ oder einer Biegeverformungsbewegung und/ oder eines Grads der Biegeverformung eines Lagerungsschaftabschnitts (SA) eines Lagerungsschafts (3);
- Vergleich des Biegeverformungszustands und/ oder der Biegeverformungsbewegung, und/ oder des Grads der Biegeverformung des Lagerungsschaftabschnitts (SA) mit Referenzdaten;
- Bestimmen eines Verschleißgrades basierend auf dem Vergleich;
- Ausgabe des Verschleißgrades.

14. Verfahren nach Anspruch 13, wobei das Verfahren weiterhin den Schritt beinhaltet:
- Ausgabe einer Lebensdauerprädiktion und/ oder einer Austauschempfehlung für das medizinische Schaftwerkzeug (9).

15. Verfahren nach Anspruch 13 oder 14, wobei das Verfahren weiterhin den Schritt beinhaltet:
- Anpassen von Antriebsparametern des medizinischen Schaftwerkzeugs (9) basierend auf dem bestimmten Verschleißgrad des medizinischen Schaftwerkzeugs (9).
